# EUROPÄISCHE PATENTSCHRIFT

(11) **EP 2 045 233 B1**
(45) Veröffentlichungstag und Bekanntmachung des Hinweises auf die Patenterteilung: **28.11.2012**
(21) Anmeldenummer: 08012181.7
(22) Anmeldetag: 05.07.2008
(51) Int. Cl.: C07C 67/60, C07C 69/28

(54) **Verfahren zur Farbverbesserung von Oligoglykolcarbonsäureestern**
Method for improving the colour of oligoglycol carboxylic acid esters
Procédé d'amélioration de la couleur d'esters oligoglycoliques d'acide carboxylique

(30) Priorität: 04.10.2007 DE 102007047345
(43) Veröffentlichungstag der Anmeldung: 08.04.2009
(73) Patentinhaber: Celanese Chemicals Europe GmbH, 61476 Kronberg (DE)
(72) Erfinder: Berger, Günther, 65824 Schwalbach (DE); Haase, Wolfgang, 65830 Kriftel (DE); Strutz, Heinz Dr., 47445 Moers (DE); Trautmann, Heinz, 65220 Bad Camberg (DE)

(56) Entgegenhaltungen:
- BE-A- 519 660
- DE-A1- 3 836 093
- US-A- 3 891 694
- IUPAC: "oligomer molecule", IUPAC Compendium of Chemical Terminology, 1997, Retrieved from the Internet: URL:http://old.iupac.org/goldbook/O04286.p df [retrieved on 2011-02-08]

## Beschreibung

Die Erfindung betrifft ein Verfahren zur Farbverbesserung von Diestern von Oligoglykolen mit gesättigten aliphatischen Monocarbonsäuren durch Einleiten von Sauerstoff oder Sauerstoff enthaltenden Gasen in den Rohester und anschließender schonender Destillation.

Oligoglykolcarbonsäureester sind Esterverbindungen, die aus Diolen bzw. Etherdiolen als Alkoholkomponente und aus Monocarbonsäuren als Säurekomponente aufgebaut sind.

Oligoglykolcarbonsäureester finden als Weichmacher für Kunststoffe, Beschichtungsmittel, Dichtungsmassen, Kautschuck- und Gummiartikel eine weite Verwendung. Sie treten mit hochpolymeren thermoplastischen Stoffen, ohne chemisch zu reagieren, vorzugsweise durch ihr Löse- und Quellvermögen in physikalische Wechselwirkung. Hierzu bildet sich ein homogenes System aus, dessen thermoplastischer Bereich gegenüber den ursprünglichen Polymeren zu niedrigeren Temperaturen verschoben ist. Durch gezielte Wahl und Dosierung bestimmter Oligoglykolcarbonsäureester lassen sich die mechanischen Eigenschaften, wie Härte, Kälteflexibilität und Festigkeit, der Polymeren je nach Einsatzgebiet genau einstellen.

Um Oligoglykolcarbonsäureestern möglichst weite Anwendungsgebiete zu eröffnen, müssen sie eine Reihe von Kriterien erfüllen. Im Idealfall sollten sie geruchlos, farblos, licht-, kälte- und wärmebeständig sein. Überdies erwartet man, dass sie unempfindlich gegenüber Wasser, schwer brennbar und wenig flüchtig sind sowie die Gesundheit nicht schädigen. Weiterhin soll ihre Herstellung einfach sein und, um ökologischen Erfordernissen zu genügen, unter Vermeidung von Abfallstoffen, wie nicht weiterverwertbaren Nebenprodukten und Schadstoffe enthaltenden Abwässern, erfolgen.

Oligoglykolcarbonsäureester enthalten als Alkoholkomponente Diole bzw. Etherdiole, insbesondere Ethylenglykol, Diethylenglykol, Triethylenglykol, und Tetraethylenglykol. Oligoglykolcarbonsäureester lassen sich auf verschiedene Weise herstellen. Neben der Umsetzung von Alkohol und Säure, gegebenenfalls in Gegenwart saurer Katalysatoren, werden in der Praxis noch weitere Prozesse angewandt, z.B. die Umsetzung von Diol mit einem Säurehalogenid, die Umesterung eines Carbonsäureesters mit einem Diol und die Addition von Ethylenoxid an die Carbonsäure (Ethoxylierung). Im industriellen Maßstab werden Oligoglykolcarbonsäureester durch direkte Umsetzung von Diol mit Carbonsäure oder durch Ethoxylierung erhalten, wobei der direkten Umsetzung von Diol mit Carbonsäure der Vorzug gegeben wird, denn dieses Verfahren kann ohne besonderen Aufwand in einfachen, kommerziell leicht zugänglichen Apparaten durchgeführt werden und es liefert chemisch einheitliche Produkte. Demgegenüber erfordert die Ethoxylierung umfangreiche und kostenintensive technische Mittel und zudem hohe Sicherheitsmaßnahmen aufgrund des aggressiven chemischen Verhaltens von Ethylenoxid. Zudem ist die Reaktion mit Ethylenoxid nicht sehr selektiv und führt zu Gemischen von Verbindungen unterschiedlicher Kettenlänge.

Die unmittelbare Veresterung von Alkoholen mit Carbonsäuren gehört zu den Grundoperationen der organischen Chemie.

Um die Reaktion in Richtung des Oligoglykolcarbonsäureesters zu lenken, ist es zweckmäßig, das Reaktionswasser kontinuierlich zu entfernen. Aus Fatty Acids in Industry (1989), Kap. 9, Polyoxyethylene Esters of Fatty Acids, sind geeignete Maßnahmen bekannt, wie die Azeotropdestillation in Gegenwart eines mit Wasser nicht mischbaren Lösungsmittels, das Erhitzen des Reaktionsgemisches unter Durchleiten eines Inertgases, die Durchführung der Umsetzung unter Vakuum oder in Anwesenheit eines Trockenmittels.Die Reaktion kann in Abwesenheit von Katalysatoren erfolgen, erfordert dann aber höhere Temperaturen und längere Reaktionszeiten. Beide Reaktionsbedingungen können durch Einsatz saurer Katalysatoren gemildert werden. Neben Schwefelsäure sind organische Säuren wie p-Toluolsulfonsäure sowie Kationenaustauscher vom Polystyrolsulfonsäure-Typ die bevorzugten Katalysatoren.

Ein wichtiges Qualitätskriterium für den Einsatz von Oligoglykolcarbonsäureestern als Weichmacher ist ihre Farbzahl. So ist z.B. für die Herstellung von transparenten Kunststoffartikeln, wie von transparenten Folien, die Verwendung möglichst farbloser Oligoglykolcarbonsäureester notwendig. Die Einstellung einer für viele Anwendungsgebiete notwendigen niedrigen Farbzahl durch konventionelle Destillation über eine Kolonne ist schwierig und im Allgemeinen wenig erfolgreich. Über eine Farbvertiefung hinaus kann eine längere thermische Belastung in einer konventionellen Destillation über Kolonne auch zu einer vermehrten Esterspaltung führen. Die damit verbundene Erhöhung des Säurewertes des Esters ist für viele Anwendungen nachteilig.

Durch den nachträglichen Zusatz von Bleichmitteln oder von Klärungsmitteln, wie zum Beispiel von Aktivkohle, lässt sich die Farbzahl verbessern. Diese Maßnahmen machen jedoch einen zusätzlichen Aufarbeitungs- bzw. Filtrationsschritt erforderlich, der zudem noch zu Produktverlusten führt und Entsorgungsprobleme für das verbrauchte Absorptionsmittel aufwirft. Der Zusatz von Bleichmitteln, die häufig nur schwer oder gar nicht mehr aus dem Oligoglykolcarbonsäureester abtrennbar sind, können die anwendungstechnischen Eigenschaften beeinträchtigen.

Hellfarbige Carbonsäureester erhält man nach DE 197 20257 C1 auch dadurch, dass die katalytische Veresterungsreaktion in Gegenwart von Phosphinsäure und Zinnsalzen durchgeführt wird. Führt man die Veresterung nach diesem Verfahren durch, so lassen sich die Phosphorverbindungen in Gegenwart der Zinnsalze leicht auswaschen und es wird ein Ester mit einem nur geringen, aber deutlich nachweisbaren Phosphorgehalt erhalten. Dieser kann zu anwendungstechnischen Problemen beim Einsatz der Weichmacher führen.

Eine Nächreinigung von Esterverbindungen mit Wasserstoffperoxid ist ein aus der Technik bekanntes Verfahren (vgl. z.B. H. Suter, Phthalsäureanhydrid und seine Verwendung, Dr. Dietrich Steinkopf Vertag, Darmstadt 1972, Seite 80). Bei diesem Verfahren erfordert der Umgang mit dem aggressiven Wasserstoffperoxid gesonderte Apparate und Behälter sowie besondere Sicherheitsmaßnahmen. Zudem muß das bei der Bleiche mit Wasserstoffperoxid eingetragene und aus dem Zerfall des Wasserstoffperoxids entstehende Wasser in einem weiteren Trocknungsschritt entfernt werden, um der Spezifikationsanforderung vieler Anwendungen des Weichmachers zu genügen.

Darüber hinaus wird in BE 519 660 A die Farbverbesserung von Weichmachem, z. B. Triethylenglykoldi-2-ethylhexanoat, durch Behandlung mit Wasserstoff offenbart. US-A-3 891 694 beschreibt die Entfärbung von Weichmachern, wie z. B. Adipinsäuredioctylester durch Erhitzen mit Persäure (z.B. Peressigsäure) im Vakuum.

Nachteilig bei den bekannten Verfahren ist zum einen der Eintrag von Hilfsstoffen, die nur bis zu einem gewissen Maße und nicht vollständig aus dem Oligoglykolcarbonsäureester herausgewaschen werden können oder zum Anderen das Arbeiten mit Wasserstoffperoxid, das zusätzliche Sicherheits- und Folgemaßnahmen erfordert.

Die Aufgabe der Erfindung hat darin bestanden, ein einfaches Verfahren zur Farbverbesserung von Diestern von Oligoglykolen mit gesättigten aliphatischen Monocarbonsäuren zur Verfügung zu stellen, das solche Diester mit niedriger Farbzahl liefert. Besonderer Wert wird in diesem Zusammenhang darauf gelegt, dass der Prozess mit einfachen technischen Mitteln realisiert werden kann, lange Betriebszeiten sicherstellt, über die Betriebsdauer Produkte mit gleichbleibend geringer Farbzahl liefert und möglichst keine Koppelprodukte erzeugt.

Die Erfindung besteht in einem Verfahren zur Farbverbesserung von Diestern von Oligoglykolen, die durch Veresterung gesättigter, acyclischer, linearer oder verzweigter, aliphatischer Monocarbonsäuren mit 3 bis 20 Kohlenstoffatomen im Molekül mit einem Monoglykol oder einem oligomeren Glykol erhalten werden. Es ist dadurch gekennzeichnet dass der unter vermindertem Druck stehende, rohe Oligoglykolcarbonsäurediester bei einer Temperatur von 20 bis 230°C durch Einleiten von Sauerstoff oder Sauerstoff enthaltenden Gasen bis auf maximal Normaldruck begast und gleichzeitig oder anschließend einer Destillation unterzogen wird.

Überraschenderweise wurde gefunden, dass sich die Farbzahl des Rohesters deutlich verbessern lässt, wenn der Rohester nicht - wie für viele organische Verbindungen zu erwarten bzw. notwendig - unter möglichst inerten Bedingungen wie Vakuum oder Inertgas gehalten wird, sondern das aufgeheizte und unter Vakuum stehende Rohprodukt durch Zugabe von Sauerstoff oder Sauerstoff enthaltenden Gasen, wie zum Beispiel Luft, bis auf maximal Normaldruck begast und erst dann destilliert wird.

Eine Farbzahlverbesserunq des Rohesters erzielt man ebenfalls, wenn man während der Vakuumdestillation in geringem Umfang den Zutritt von Sauerstoff oder Sauerstoff enthaltenden Gasen, wie z. B. Luft, zulässt. Dabei leitet man das Gas dosiert als "Falschluft" durch die eigentliche Destillationsapparatur.

Überraschend ist auch, dass die gefundene Methode der oxidativen Farbverbesserung sich nicht nachteilig auf die anwendungstechnisch bedeutsame Peroxid-Zahl des Oligoglykolcarbonsäurediesters und auf seinen Wassergehalt auswirkt.

Die Durchführung des erfindungsgemäßen Verfahrens ist nicht auf eine bestimmte Apparatur beschränkt. Die Begasung mit Sauerstoff oder einem Sauerstoff enthaltenden Gas stellt keine besonderen apparativen Anforderungen, sofern eine Verteilung des eingebrachten Gases möglich ist. Für die sich anschließende (oder ggf. gleichzeitig ablaufende) Destillation des beschriebenen erfindungsgemäßen Verfahrens lassen sich alle Apparate verwenden, die dem Fachmann bekannt und handelsüblich sind, um hochsiedende Flüssigkeiten, deren Siedepunkte selbst bei Drücken von kleiner 1 mbar bei hohen Temperaturen, beispielsweise über 150°C liegen, destillativ zu reinigen. Bevorzugte Apparate, in denen das erfindungsgemäße Verfahren durchgeführt werden kann, sind Kurzwegverdampfer oder Dünnschichtverdampfer mit einer gerührten oder/und umgepumpten Destillationsvorlage zur Begasung.

Bei dem erfindungsgemäßen Verfahren zur Farbverbesserung von gefärbten, rohen Oligoglykolcarbonsäureestern gibt man zu dem bei der Veresterung angefallenen und unter einem Druck < 100 mbar befindlichen Rohprodukt bei einer Temperatur von im allgemeinen 20 bis 230°C, vorzugsweise 60 bis 190°C und insbesondere 120 bis 150°C stufenweise Sauerstoff oder Sauerstoff enthaltende Gase, vorzugsweise Luft, bis der Druck in der Destillationsvorlage auf maximal Normaldruck, vorzugsweise bis auf 500 mbar, angestiegen ist. Die Temperatur bei der Dosierung und die Dosiermenge des einzuleitenden Gases hängen von der Art und den Eigenschaften des zu behandelnden Rohesters ab.

Das Einleiten des Gases kann durch einfaches Belüften der Destillationsvorlage erfolgen.

Es hat sich aber als zweckmäßig erwiesen, das für die Behandlung verwendete Gas über ein Tauchrohr direkt durch den Rohester perlen zu lassen. Besonders zweckmäßig ist es, das Tauchrohr mit einer Fritte zu versehen, um eine möglichst feine Verteilung des verwendeten Gases in der Flüssigkeit zu erzielen. Während der Behandlung mit Sauerstoff oder einem Sauerstoff enthaltenden Gas wird der Rohester vorzugsweise gerührt oder umgepumpt, um auch dadurch einen optimalen Kontakt des einströmenden Gases mit der den Rohester anfärbenden Komponente zu erreichen.

Die sich anschließende Destillation des so behandelten Rohesters erfolgt anschließend unter möglichst schonenden Bedingungen hinsichtlich Temperaturbelastung und/oder Verweilzeit. Für die eigentliche Destillation sind daher solche Destillationsapparate geeignet, die eine schonende Destillation erlauben. Dies sind vorzugsweise Apparate, die auch im Produktionsmaßstab einen Druck von kleiner 5 mbar zulassen und eine möglichst geringe bzw. kurze Temperaturbelastung des vorbehandelten Esters erlauben. Die konkreten Temperatur- und Druckbedingungen richten sich nach dem jeweiligen zu reinigenden Oligoglykolcarbonsäurediester und sind durch Routineversuche leicht zu ermitteln.

In einer weiteren Ausgestaltung des erfindungsgemäßen Verfahrens führt man die erfindungsgemäße Behandlung des Rohesters während der Destillation durch. Die dabei in die eigentliche Destillationsapparatur einzuleitenden Gasmengen sind nur gering und im allgemeinen so bemessen, daß die Destillation weiterhin bei Drücken unterhalb von 500 mbar und somit unter möglichst schonender Temperaturbelastung und geringer Verweilzeit betrieben werden kann. Die konkreten Temperatur- und Druckbedingungen richten sich nach dem jeweiligen zu reinigenden Oligoglykolcarbonsäurediester.

Insbesondere sind Dünnschichtverdampfer oder Kurzwegverdampfer geeignet.

Die Destillation kann sowohl kontinuierlich wie diskontinuierlich durchgeführt werden, unabhängig davon, ob die Destillation im Anschluß oder während der erfindungsgemäßen Begasung erfolgt.

Die Menge des Destillats liegt im Allqemeinen bei 30 bis 95 Gew.-%, vorzugsweise bei 50 bis 90 Gew.-% und insbesondere bei 75 bis 85 Gew.-% vom Einsatzprodukt. Das angefallene Sumpfprodukt kann einer erneuten Destillation unterzogen werden, wobei das dabei anfallende Destillat erneut der erfinderischen Behandlung mit Sauerstoff oder Sauerstoff enthaltenden Gasen unterzogen werden kann, aber oftmals nicht muss, da das Destillat aus dem angefallenen Sumpfprodukt schon eine zufriedenstellende Farbzahl aufweist.

Auf diese Weise erhält man ca. 92 Gew.-% des eingesetzten Rohesters als hellfarbiges Destillat, das im Allgemeinen eine Hazen-Farbzahl von kleiner 20 Hazen (HZ) aufweist. Die Bestimmung nach Hazen ist eine gebräuchliche Methode zur Farbcharakterisierung transparenter Flüssigkeiten (Römpps Chemie Lexikon, 9. Auflage, Bd. 2, Seite 1306).

Der verbleibende Sumpf kann bei einer Folgedestillation wiedereingesetzt werden, so dass die Gesamtausbeute über einen längeren Zeitraum über 95 Gew.-% betragen kann.

Der Vorteil des erfindungsgemäßen Verfahrens liegt darin, dass es in ohnehin für konventionelle destillative Aufarbeitungen vorhandenen und genutzten Apparaten ohne großen zusätzlichen apparativen Aufwand durchgeführt werden kann. Zudem führt die Farbverbesserung durch Begasung mit Sauerstoff oder einem Sauerstoff enthaltenden Gas mit anschließender oder bei gleichzeitiger Destillation nicht zu Koppelprodukten, die nachträglich in einem weiteren Schritt noch zu entfernen sind. Die zur Herstellung der aliphatischen Oligoglykolcarbonsäureester eingesetzten Monocarbonsäuren sind gesättigte, acyclische, lineare oder verzweigte, aliphatische Carbonsäuren. Beispiele für Carbonsäuren als Säurekomponente für Oligoglykolcarbonsäureester sind n-Propionsäure, n-Buttersäure, Isobuttersäure, n-Pentansäure, 2-Methylbuttersäure, 3-Methylbuttersäure, 2-Methylpentansäure, 2-Ethylbuttersäure, n-Hexansäure, n-Heptansäure, 2-Methylhexansäure, 2-Ethylhexansäure, n-Nonansäure, 2-Methyloctansäure, Isononansäure, n-Dekansäure, 2-Methylundecansäure, Isoundecancarbonsäure und Isotridecancarbonsäure.

Die als Ausgangsstoffe verwendeten Mono-, Di-, Tri- und Tetraethylenglykole sind industriell produzierte Chemikalien. Basissubstanz zu ihrer Herstellung ist Ethylenoxid, aus dem man Monoethylenglykol durch Erhitzen mit Wasser unter Druck in großtechnischem Maßstab gewinnt. Diethylenglykol erhält man durch Ethoxylierung aus Ethylenglykol. Triethylenglykol fällt, wie Tetraethylenglykol, als Nebenprodukt bei der Hydrolyse von Ethylenoxid zur Herstellung von Monoethylenglykol an. Beide Verbindungen können aber auch durch Umsetzung von Monoethylenglykol mit Ethylenoxid synthetisiert werden.

Besonders geeignet ist das erfindungsgemäße Reinigungsvefahren für solche Oligoglykolcarbonsäurediester, die durch Veresterung gesättigter aliphatischer C_{S}- bis C₁₃-Monocarbonsäuren, insbesondere von C₄- bis C₉-Monocarbonsäuren, wie beispielsweise 2-Methylpentansäure oder n-Heptansäure mit Mono-, Di-, Tri- oder Tetraethylenglykol erhalten werden.

Die Veresterung von Carbonsäuren mit Glykolen ist ein aus dem Stand der Technik bekanntes Verfahren. Die Veresterung kann in Abwesenheit eines

Katalysators erfolgen, mit dem Vorteil daß man vermeidet, dem Reaktionsgemisch Fremdstoffe zuzuführen. Allerdings muß man dann im Allgemeinen höhere Reaktionstemperaturen einhalten, um die Umsetzung mit ausreichend hoher Geschwindigkeit ablaufen zu lassen. Ferner kann eine übermäßige thermische Belastung zu einer Schädigung des Esters und einer Farbvertiefung führen. Setzt man keinen zusätzlichen Katalysator ein, um die Reaktionsgeschwindigkeit zu erhöhen, so empfiehlt es sich, einen Überschuß an Carbonsäure zu verwenden, die gleichzeitig als Reaktionskomponente für das Glykol und als Katalysator dient, Häufig setzt man jedoch auch die üblichen Veresterungskatalysatoren, wie Schwefelsäure, p-Toluolsulfonsäure, Methansulfonsäure oder Phosphorsäure hinzu.

Die nach den bekannten Verfahrenschritten erhaltenen rohen Oligoglykolcarbonsäurediester sind in der Regel hellgelbe bis hellbraune, bei Raumtemperatur viskose Flüssigkeiten, wobei lange Veresterungszeiten, hohe Veresterungstemperaturen oder hohe Katalysatorkonzentrationen im Allgemeinen zu stärker gefärbten Rohprodukten führen.

Die Veresterung kann mit stöchiometrischen Mengen Alkohol und Säure vorgenommen werden. Vorzugsweise läßt man das Glykol mit überschüssiger Säure reagieren, um eine möglichst vollständige Umsetzung des Diols zu erreichen. Zudem läßt sich ein Säureüberschuß destillativ leichter aus dem Rohgemisch entfernen.

Zur Entfernung des Reaktionswassers kann man einen Azeotropbildner zusetzen. Als Azeotropbildner eignen sich inerte Lösungsmittel, wie z.B. Toluol. Aber auch überschüssige Carbonsäure kann als Azeotropbildner fungieren. Durch Auffangen und Auftrennen des abdestillierten Azeotrop-Wassergemischs läßt sich in einfacher Weise der Fortgang der Reaktion verfolgen. Der abgetrennte Azeotropbildner wird anschließend ohne weitere Reinigung in den Veresterungsprozeß zurückgefahren.

Weitere Verfahrenvarianten bestehen darin, das Reaktionswasser unter Durchleiten eines Inertgases, unter Anlegen eines Unterdrucks oder durch Zugabe eines Trockenmittels zu entfernen.

Im Allgemeinen wird der Veresterungsprozeß bei Temperaturen von etwa 100 bis 250°C und bei Drücken von Normaldruck bis zu 1 mbar durchgeführt.

Das nach der Beendigung der Umsetzung und ggf. nach der destillativen Entfernung des Carbonsäureüberschusses bzw. nach der Katalysatorabtrennung anfallende Rohgemisch ist in aller Regel von der chemischen Zusammensetzung her bereits sehr sauber und bedarf nur noch einer Farbverbesserung.

Die gefärbten Oligoglykolcarbonsäurediester werden anschließend dem erfindungsgemäßen Reinigungsverfahren unterzogen. Für die erfolgreiche Durchführung des erfindungsgemäßen Reinigungsverfahrens spielt es keine Rolle, nach welcher gewählten Veresterungsvariante der spezielle Oligoglykolcarbonsäurediester hergestellt wurde.

Die nach dem erfindungsgemäßen Vorfahren gereinigten Oligoglykolcarbonsäurediester fallen als hellfarbige Flüssigkeiten an, die sich als Weichmacher für alle gängigen hochpolymeren thermoplastischen Stoffe eignen. Besonders bewährt haben sie sich als Zusatz zu Poyvinylbutyral, das mit Oligoglykolcarbonsäurediestem versetzt als Zwischenschicht zur Herstellung von Mehrschichten- oder Verbundgläsern verwendet wird.

In den folgenden Beispielen wird das erfindungsgemäße Verfahren näher erläutert. Es ist jedoch nicht auf die beschriebene Ausführungsform beschränkt.

### Herstellung von Triethylenglykol-di-heptanoat:

In einem 101-Mehrhalskolben (ausgerüstet mit Rührer, N2-Einperlung, Vakuumanschluß, Kolonne und Wasserabscheider) werden unter Stickstoff 3.26 kg (25.1 mol) Heptansäure (Reinheit: 99%), 1.1 kg aus einem vorhergehenden Ansatz rückgewonnene Heptansäure (Mischung aus Heptansäure und Triethylenglykol-di-heptanoat mit 53% Anteil an Heptansäure; 4.5 mol)) und 2.16 kg (14.4 mol) Triethylenglykol (Reinheit: 99,7%) gemischt und auf Rückflußtemperatur erhitzt. Das Reaktionswasser wird im Abscheider von der organischen Phase getrennt und ausgeschleust; die organische Phase wird in den Reaktionskolben zurückgeführt. Nach Abscheiden von mehr als der Hälfte des berechneten Reaktionswassers werden weitere 0.46 kg (3.5 mol) Heptansäure nachgeführt. Nachdem das Reaktionswasser weitgehend ausgeschleust ist, wird die überschüssige Heptansäure im Gemisch mit Produkt bei einem kontinuierlich vermindertem Druck von bis < 1 mbar und einer Temperatur von 180 - 220°C abdestilliert, bis im Sumpf ein Säuregehalt < 0.02% erreicht ist. Das rohe Triethylenglykol-di-heptanoat verbleibt als gelblich gefärbtes Produkt im Sumpf. Rohausbeute: 5.3 kg

### Reinigung von rohem Triethylenglykol-di-heptanoat:

In einem 10I-Mehrhalskolben (ausgerüstet mit Rührer, N2- bzw. Luft-Einperlung, Vakuumanschluß) werden 5.3 kg rohes Triethylenglykol-di-heptanoat unter Stickstoff eingefüllt und anschließend unter Rühren bei Raumtemperatur auf einen Druck von ca. 100 mbar gesetzt. Anschließend wird das Rohprodukt in einem Kurzwegverdampfer (0.01 m²) bei einem Druck < 1 mbar und einer Heizmanteltemperatur des Kurzwegverdampfers > 195° C bei einem Rohprodukt-Zulauf von 0.45 kg/h und einem Destillat-zu-Sumpfverhältnis von 4 zu 1 destilliert. Das Destillat zeigt in einer Farbzahlmessung eine Hazen-Zahl > 35 Hz. Destillat und Sumpf werden in das Vorlagegefäß kontinuierlich zurückgeschleust.

Anschließend wird im Vorlagegefäß unter Rühren der Rohester auf eine Temperatur von ca. 120 - 130°C erhitzt und der Druck in mehreren Schritten durch Einperlen von Luft bis auf 500 mbar erhöht. Die Farbzahl im Destillat-Ablauf der Kurzwegdestillation verbessert sich innerhalb kurzer Zeit auf 20 Hz. Destillat- und Sumpfablauf werden dann auf getrennte Vorlagen gefahren. Nach Durchschleusen der 5.3 kg des rohen Triethylenglykol-di-heptanoats wird der Sumpf einer wiederholten Redestillation unterworfen; der dann verbleibende Destillatrückstand kann in einer Folgedestillation miteingesetzt werden.

Ausbeute: 4.9 kg (Farbzahl: 20 Hz; Reinheit: > 97.5% gaschromatographisch ermittelt; Peroxid: < 1 meq/kg; Säuregehalt: 0.02%; Wassergehalt: 0.05%)

## Patentansprüche

1. Verfahren zur Farbverbesserung von Estern von Oligoglykolen, die durch Veresterung gesättigter acyclischer, linearer oder verzweigter, aliphatischer Monocarbonsäuren mit 3 bis 20 Kohlenstoffatomen im Molekül mit einem Monoglykol oder einem oligomeren Glykol erhalten werden, **dadurch gekennzeichnet, dass** der unter vermindertem Druck stehende, rohe Oligoglykolcarbonsäureester bei einer Temperatur von 20 bis 230 °C durch Einleiten von Sauerstoff oder Sauerstoff enthaltenden Gasen bis auf maximal Normaldruck begast und gleichzeitig oder anschließend einer Destillation unterzogen wird.

2. Verfahren nach Anspruch 1, **dadurch gekennzeichnet, dass** der rohe Oligoglykolcarbonsäureester bei einer Temperatur von 60 bis 190°C durch Einleiten von Sauerstoff oder Sauerstoff enthaltenden Gasen begast wird.

3. Verfahren nach Anspruch 1, **dadurch gekennzeichnet, dass** der rohe Oligoglykolcarbonsäureester bei einer Temperatur von 120 bis 150°C durch Einleiten von Sauerstoff oder Sauerstoff enthaltenden Gasen begast wird.

4. Verfahren nach einem der Ansprüche 1 bis 3, **dadurch gekennzeichnet, dass** durch Einleiten von Sauerstoff oder Sauerstoff enthaltenden Gasen bis auf einen Druck von 500 mbar begast wird.

5. Verfahren nach einem der Ansprüche 1 bis 4, **dadurch gekennzeichnet, dass** man Oligoglykolcarbonsäureester auf Basis von Monocarbonsäuren mit fünf bis dreizehn Kohlenstoffatomen im Molekül und Mono-, Di-, Tri- oder Tetraethylenglykolen reinigt.

6. Verfahren nach Anspruch 5, **dadurch gekennzeichnet, dass** man Diester des Monoethylenglykols mit jeweils einer der folgenden Carbonsäuren: n-Propionsäure, n-Buttersäure, n-Pentansäure, n-Hexansäure, n-Heptansäure, n-Nonansäure, n-Dekansäure reinigt.

7. Verfahren nach Anspruch 5, **dadurch gekennzeichnet, dass** man Diester des Diethylenglykols mit jeweils einer der folgenden Carbonsäuren: n-Propionsäure, n-Buttersäure, n-Pentansäure, n-Hexansäure, n-Heptansäure, n-Nonansäure, n-Dekansäure reinigt.

8. Verfahren nach Anspruch 5, **dadurch gekennzeichnet, dass** man Diester des Triethylenglykols mit jeweils einer der folgenden Carbonsäuren: n-Propionsäure, n-Buttersäure, n-Pentansäure, n-Hexansäure, n-Heptansäure, n-Nonansäure, n-Dekansäure reinigt.

9. Verfahren nach Anspruch 5, **dadurch gekennzeichnet, dass** man Diester des Tetraethylenglykols mit jeweils einer der folgenden Carbonsäuren: n-Propionsäure, n-Buttersäure, n-Pentansäure, n-Hexansäure, n-Heptansäure, n-Nonansäure, n-Dekansäure reinigt.

10. Verfahren nach Anspruch 5, **dadurch gekennzeichnet, dass** man Diester des Monoethylenglykols mit jeweils einer der folgenden Carbonsäuren:
Isobuttersäure, 2-Methylbuttersäure, 3-Methylbuttersäure, 2-Methylpentansäure, 2-Ethylbuttersäure, 2-Methylhexansäure, 2-Ethylhexansäure, 2-Methyloctansäure, Isononansäure, 2-Methylundecansäure, Isoundecancarbonsäure, Isotridecancarbonsäure reinigt.

11. Verfahren nach Anspruch 5, **dadurch gekennzeichnet, dass** man Diester des Diethylenglykols mit jeweils einer der folgenden Carbonsäuren:
Isobuttersäure, 2-Methylbuttersäure, 3-Methylbuttersäure, 2-Methylpentansäure, 2-Ethylbuttersäure, 2-Methylhexansäure, 2-Ethylhexansäure, 2-Methyloctansäure, Isononansäure, 2-Methylundecansäure, Isoundecancarbonsäure, Isotridecancarbonsäure reinigt.

12. Verfahren nach Anspruch 5, **dadurch gekennzeichnet, dass** man Diester des Triethylenglykols mit jeweils einer der folgenden Carbonsäuren:
Isobuttersäure, 2-Methylbuttersäure, 3-Methylbuttersäure, 2-Methylpentansäure, 2-Ethylbuttersäure, 2-Methylhexansäure, 2-Ethylhexansäure, 2-Methyloctansäure, Isononansäure, 2-Methylundecansäure, Isoundecancarbonsäure, Isotridecancarbonsäure reinigt.

13. Verfahren nach Anspruch 5, **dadurch gekennzeichnet, dass** man Diester des Tetraethylenglykols mit jeweils einer der folgenden Carbonsäuren:
Isobuttersäure, 2-Methylbuttersäure, 3-Methylbuttersäure, 2-Methylpentansäure, 2-Ethylbuttersäure, 2-Methylhexansäure, 2-Ethylhexansäure, 2-Methyloctansäure, Isononansäure, 2-Methylundecansäure, Isoundecancarbonsäure, Isotridecancarbonsäure reinigt.

14. Verfahren nach Anspruch 1, **dadurch gekennzeichnet, dass** ein Dünnschichtverdampfer zur destillativen Aufarbeitung verwendet wird.

15. Verfahren nach Anspruch 1, **dadurch gekennzeichnet, dass** ein Kurzwegverdampfer zur destillativen Aufarbeitung verwendet wird.

16. Verfahren nach einem der Ansprüche 1 bis 4, **dadurch gekennzeichnet, dass** mit Luft begast wird.

## Claims

1. Process for improving the colour of esters of oligoglycols which are obtained by esterifying saturated acyclic, linear or branched, aliphatic monocarboxylic acids having 3 to 20 carbon atoms in the molecule with a monoglycol or an oligomeric glycol, **characterized in that** the crude oligoglycol carboxylate under reduced pressure is sparged up to a maximum of standard pressure by introduction of oxygen or oxygen-containing gases at a temperature of 20 to 230°C, and simultaneously or subsequently subjected to a distillation.

2. Process according to Claim 1, **characterized in that** the crude oligoglycol carboxylate is sparged by introduction of oxygen or oxygen-containing gases at a temperature of 60 to 190°C.

3. Process according to Claim 1, **characterized in that** the crude oligoglycol carboxylate is sparged by introduction of oxygen or oxygen-containing gases at a temperature of 120 to 150°C.

4. Process according to any of Claims 1 to 3, **characterized in that** sparging is effected up to a pressure of 500 mbar by introduction of oxygen or oxygen-containing gases.

5. Process according to any of Claims 1 to 4, **characterized in that** oligoglycol carboxylates based on monocarboxylic acids having five to thirteen carbon atoms in the molecule and mono-, di-, tri- or tetraethylene glycols are purified.

6. Process according to Claim 5, **characterized in that** diester of monoethylene glycol with one of the following carboxylic acids in each case: n-propionic acid, n-butyric acid, n-pentanoic acid, n-hexanoic acid, n-heptanoic acid, n-nonanoic acid, n-decanoic acid is purified.

7. Process according to Claim 5, **characterized in that** diester of diethylene glycol with one of the following carboxylic acids in each case: n-propionic acid, n-butyric acid, n-pentanoic acid, n-hexanoic acid, n-heptanoic acid, n-nonanoic acid, n-decanoic acid is purified.

8. Process according to Claim 5, **characterized in that** diester of triethylene glycol with one of the following carboxylic acids in each case: n-propionic acid, n-butyric acid, n-pentanoic acid, n-hexanoic acid, n-heptanoic acid, n-nonanoic acid, n-decanoic acid is purified.

9. Process according to Claim 5, **characterized in that** diester of tetraethylene glycol with one of the following carboxylic acids in each case: n-propionic acid, n-butyric acid, n-pentanoic acid, n-hexanoic acid, n-heptanoic acid, n-nonanoic acid, n-decanoic acid is purified.

10. Process according to Claim 5, **characterized in that** diester of monoethylene glycol with one of the following carboxylic acids in each case: isobutyric acid, 2-methylbutyric acid, 3-methylbutyric acid, 2-methylpentanoic acid, 2-ethylbutyric acid, 2-methyl-hexanoic acid, 2-ethylhexanoic acid, 2-methyloctanoic acid, isononanoic acid, 2-methylundecanoic acid, isoundecanecarboxylic acid, isotridecanecarboxylic acid is purified.

11. Process according to Claim 5, **characterized in that** diester of diethylene glycol with one of the following carboxylic acids in each case: isobutyric acid, 2-methylbutyric acid, 3-methylbutyric acid, 2-methylpentanoic acid, 2-ethylbutyric acid, 2-methyl-hexanoic acid, 2-ethylhexanoic acid, 2-methyloctanoic acid, isononanoic acid, 2-methylundecanoic acid, isoundecanecarboxylic acid, isotridecanecarboxylic acid is purified.

12. Process according to Claim 5, **characterized in that** diester of triethylene glycol with one of the following carboxylic acids in each case: isobutyric acid, 2-methylbutyric acid, 3-methylbutyric acid, 2-methylpentanoic acid, 2-ethylbutyric acid, 2-methyl-hexanoic acid, 2-ethylhexanoic acid, 2-methyloctanoic acid, isononanoic acid, 2-methylundecanoic acid, isoundecanecarboxylic acid, isotridecanecarboxylic acid is purified.

13. Process according to Claim 5, **characterized in that** diester of tetraethylene glycol with one of the following carboxylic acids in each case: isobutyric acid, 2-methylbutyric acid, 3-methylbutyric acid, 2-methylpentanoic acid, 2-ethylbutyric acid, 2-methyl-hexanoic acid, 2-ethylhexanoic acid, 2-methyloctanoic acid, isononanoic acid, 2-methylundecanoic acid, isoundecanecarboxylic acid, isotridecanecarboxylic acid is purified.

14. Process according to Claim 1, **characterized in that** a thin-film evaporator is used for distillative workup.

15. Process according to Claim 1, **characterized in that** a short-path evaporator is used for distillative workup.

16. Process according to any of Claims 1 to 4, **characterized in that** sparging is effected with air.

## Revendications

1. Procédé pour améliorer la couleur d'esters d'oligoglycols, qui sont obtenus par estérification d'acides monocarboxyliques aliphatiques, saturés, acycliques, linéaires ou ramifiés, comprenant 3 à 20 atomes de carbone dans la molécule avec un monoglycol ou un glycol oligomère, **caractérisé en ce que** l'ester oligoglycolique de l'acide carboxylique brut, se trouvant sous une pression réduite est soumis à l'action d'un gaz, à une température de 20 à 230°C, en introduisant de l'oxygène ou des gaz contenant de l'oxygène au maximum jusqu'à la pression normale et est simultanément ou consécutivement soumis à une distillation.

2. Procédé selon la revendication 1, **caractérisé en ce que** l'ester oligoglycolique de l'acide carboxylique brut est soumis à l'action d'un gaz à une température de 60 à 190°C en introduisant de l'oxygène ou des gaz contenant de l'oxygène.

3. Procédé selon la revendication 1, **caractérisé en ce que** l'ester oligoglycolique de l'acide carboxylique brut est soumis à l'action d'un gaz à une température de 120 à 150°C en introduisant de l'oxygène ou des gaz contenant de l'oxygène.

4. Procédé selon l'une quelconque des revendications 1 à 3, **caractérisé en ce qu'**on soumet à l'action d'un gaz par l'introduction d'oxygène ou de gaz contenant de l'oxygène jusqu'à une pression de 500 mbars.

5. Procédé selon l'une quelconque des revendications 1 à 4, **caractérisé en ce qu'**on purifie des esters oligoglycoliques d'acide carboxylique à base d'acides monocarboxyliques comprenant cinq à treize atomes de carbone dans la molécule et de monoéthylèneglycol, de diéthylèneglycol, de triéthylèneglycol ou de tétraéthylèneglycol.

6. Procédé selon la revendication 5, **caractérisé en ce qu'**on purifie des diesters du monoéthylèneglycol avec à chaque fois un des acides carboxyliques suivants : acide n-propionique, acide n-butyrique, acide n-pentanoïque, acide n-hexanoïque, acide n-heptanoïque, acide n-nonanoïque, acide n-décanoïque.

7. Procédé selon la revendication 5, **caractérisé en ce qu'**on purifie des diesters du diéthylèneglycol avec à chaque fois un des acides carboxyliques suivants : acide n-propionique, acide n-butyrique, acide n-pentanoïque, acide n-hexanoïque, acide n-heptanoïque, acide n-nonanoïque, acide n-décanoïque.

8. Procédé selon la revendication 5, **caractérisé en ce qu'**on purifie des diesters du triéthylèneglycol avec à chaque fois un des acides carboxyliques suivants : acide n-propionique, acide n-butyrique, acide n-pentanoïque, acide n-hexanoïque, acide n-heptanoïque, acide n-nonanoïque, acide n-décanoïque.

9. Procédé selon la revendication 5, **caractérisé en ce qu'**on purifie des diesters du tétraéthylèneglycol avec à chaque fois un des acides carboxyliques suivants : acide n-propionique, acide n-butyrique, acide n-pentanoïque, acide n-hexanoïque, acide n-heptanoïque, acide n-nonanoïque, acide n-décanoïque.

10. Procédé selon la revendication 5, **caractérisé en ce qu'**on purifie des diesters du monoéthylèneglycol avec à chaque fois un des acides carboxyliques suivants : acide isobutyrique, acide 2-méthylbutyrique, acide 3-méthylbutyrique, acide 2-méthylpentanoïque, acide 2-éthylbutyrique, acide 2-méthylhexanoïque, acide 2-éthylhexanoïque, acide 2-méthyloctanoïque, acide isononanoïque, acide 2-méthylundécanoïque, acide iso-undécanecarboxylique, acide isotridécanecarboxylique.

11. Procédé selon la revendication 5, **caractérisé en ce qu'**on purifie des diesters du diéthylèneglycol avec à chaque fois un des acides carboxyliques suivants : acide isobutyrique, acide 2-méthylbutyrique, acide 3-méthylbutyrique, acide 2-méthylpentanoïque, acide 2-éthylbutyrique, acide 2-méthylhexanoïque, acide 2-éthylhexanoïque, acide 2-méthyloctanoïque, acide isononanoïque, acide 2-méthylundécanoïque, acide iso-undécanecarboxylique, acide isotridécanecarboxylique.

12. Procédé selon la revendication 5, **caractérisé en ce qu'**on purifie des diesters du triéthylèneglycol avec à chaque fois un des acides carboxyliques suivants : acide isobutyrique, acide 2-méthylbutyrique, acide 3-méthylbutyrique, acide 2-méthylpentanoïque, acide 2-éthylbutyrique, acide 2-méthylhexanoïque, acide 2-éthylhexanoïque, acide 2-méthyloctanoïque, acide isononanoïque, acide 2-méthylundécanoïque, acide iso-undécanecarboxylique, acide isotridécanecarboxylique.

13. Procédé selon la revendication 5, **caractérisé en ce qu'**on purifie des diesters du tétraéthylèneglycol avec à chaque fois un des acides carboxyliques suivants : acide isobutyrique, acide 2-méthylbutyrique, acide 3-méthylbutyrique, acide 2-méthylpentanoïque, acide 2-éthylbutyrique, acide 2-méthylhexanoïque, acide 2-éthylhexanoïque, acide 2-méthyloctanoïque, acide isononanoïque, acide 2-méthylundécanoïque, acide iso-undécanecarboxylique, acide isotridécanecarboxylique.

14. Procédé selon la revendication 1, **caractérisé en ce qu'**on utilise un évaporateur à couche mince pour le traitement par distillation.

15. Procédé selon la revendication 1, **caractérisé en ce qu'**on utilise un évaporateur instantané pour le traitement par distillation.

16. Procédé selon l'une quelconque des revendications 1 à 4, **caractérisé en ce qu'**on soumet à l'action d'un gaz avec de l'air.
